# EUROPÄISCHE PATENTANMELDUNG

(11) **EP 0 654 478 A2**
(43) Veröffentlichungstag der Anmeldung: **24.05.1995**
(21) Anmeldenummer: 94117664.6
(22) Anmeldetag: 09.11.1994
(51) Int. Cl.: C07H 15/04

(54) **Verfahren zur Herstellung eines Gemisches aus Alpha- und Beta-Chlorethylglucopyranose**

(30) Priorität: 22.11.1993 DE 4339698
(71) Anmelder: WOLFF WALSRODE AG, D-29655 Walsrode (DE)
(72) Erfinder: Szablikowski, Klaus, Dr., D-29664 Walsrode (DE); Lohrie, Martin, Dr., D-29664 Walsrode (DE); Koch, Wolfgang, Dr., D-29669 Bomlitz (DE); Langer, Reinhard, D-47800 Krefeld (DE); Buysch, Hans-Josef, Dr., D-47809 Krefeld (DE)
(74) Vertreter: Braun, Rolf, Dr.

(57) **Zusammenfassung**

Verfahren zur Herstellung eines Gemisches, im wesentlichen bestehend aus α- und β-Chlorethylglucopyranose durch Umsetzung von Chlorethanol mit Glukose, wobei in 2 bis 10 Molen Chlorethanol 1 Mol Glukose bei 100 bis 130°C in Abwesenheit von Katalysatoren vorgelöst wird, wobei gegebenenfalls Wasser abdestilliert werden kann und anschließend bei 60 bis 90°C 1 bis 30 Gew.-% saurer Katalysator, bevorzugt saure anorganische und/oder organische Ionenaustauscher, bezogen auf eingesetzte Glukose, zugesetzt werden und 10 bis 600 Minuten nachgerührt wird.

## Beschreibung

Gegenstand der Erfindung ist ein besonders schonendes Verfahren zur Synthese von Chlorethylglucopyranose nach Fischer mit hoher Raumzeitausbeute.

Die einfachste Methode zur Synthese von Alkylpyranosen ist die sauerkatalysierte Umsetzung der reduzierenden Zucker mit den entsprechenden Alkoholen als Reagenz und Lösungsmittel unter Wasserabspaltung. Es werden dabei, anders als in der Zuckerchemie sonst üblich, keine teuren Hilfsreagenzien eingesetzt und es entstehen dementsprechend auch keine problematischen Zwangsanfallstoffe.

Das formal einfache Reaktionsschema der Acetalisierung des anomeren Kohlenstoffatoms in reduzierenden C₆-Zuckern ist, wie intensive Untersuchungen ergeben haben, keine Wiedergabe der im Reaktionsgemisch tatsächlich ablaufenden Prozesse ("Chemistry of the o-glykosidic bond", A.F. Bochkov and G.E. Zaikov, Pergamon Press, Oxford 1979). 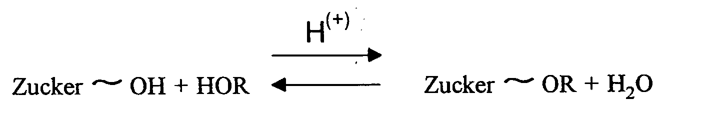
Vielmehr entsteht in der Lösung ein komplexes System aus offenkettigen, furanosiden und pyranosiden Zuckerderivaten. Die Zusammensetzung der Mischung im Gleichgewicht hängt in erster Linie vom Zucker ab und liegt fast immer auf der Seite der Pyranose.

So enthält die Gleichgewichtsmischung von Glukose in Methanol bei 35°C 1,5 % Furanoside und 98,5 % Pyranoside.

Kinetisch kontrolliert bilden sich jedoch zunächst die furanosiden Strukturen. Siehe hierzu: "Chemistry of the o-glycosidic bond", A.F. Bachkov and G.E. Zaikov, Pergamon Press, Oxford 1979, S. 11-16 und R.J. Ferrier, Topixs in Current Chemistry 14, 389 (1970).

Die Reaktion zu den thermodynamisch begünstigten Pyranosen läuft sehr langsam und führt zu einer Produktmischung der anomeren Furanoside und Pyranoside. Als Katalysatoren werden lösliche Säuren und Ionentauscherharze beschrieben, wie z.B. von G.N. Bollenbach et al. in Methods in Carbohydrate Chemistry, Vol. 2, Academic Press, New York and London, 1963, S. 228. Auch der Entzug des Reaktionswassers aus dem Gleichgewichtigsystem mit Zeolithen ist bekannt, wie er von M.B. Kozikowski et al. in Roczniki Chemii, 47, 1899 (1973) beschrieben wird.

Der Stand der Technik zur Synthese von Chlorethylglucopyranose wird in US 4 719 272, Bsp. 21, beschrieben. Dort werden 80 g Dextrose (0,5 Mol) in 148 ml (2,2 Mol) Chlorethanol mit 20 g Dowex 50 W-X8 in der H⁺-Form bei 90°C gerührt, bis die Glukose gelöst ist und dann 16 Stunden bei 60°C gehalten.

Nach dem Stand der Wissenschaft ist zu vermuten, daß die niedrigen Temperaturen und die lange Reaktionszeit zum Einstellen des Pyranosid-Gleichgewichts nötig sind. Bei höheren Temperaturen bilden Zucker i.a. farbige Nebenprodukte, die vermieden werden sollen.

Überraschenderweise wurde gefunden, daß die Chlorethylglucopyranose-Synthese bedeutend rascher durchgeführt werden kann, wenn man die Glukose bei relativ hoher Temperatur ohne Katalysator in Chlorethanol vorlöst, wobei gegebenenfalls Wasser abdestilliert werden kann, um dann bei niedrigerer Temperatur durch Zugabe von saurem Ionentauscher die Reaktion zu vervollständigen. Dabei wird in deutlich kürzerer Zeit mit deutlich weniger Katalysator nur sehr schwach verfärbtes Material erhalten.

Gegenstand der Erfindung ist ein Verfahren zur Herstellung von Chlorethylglucopyranose, welches dadurch gekennzeichnet ist, daß in 2 bis 10, bevorzugt 3 bis 8, besonders bevorzugt 4 bis 6 Molen Chlorethanol 1 Mol Glukose bei 100 bis 130°C, bevorzugt 110 bis 120°C in Abwesenheit von zusätzlichen Katalysatoren vorgelöst wird, wobei gegebenenfalls Wasser abdestilliert wird, und anschließend bei 60 bis 90°C, bevorzugt bei 70 bis 80°C, 1 bis 30, bevorzugt 2 bis 20, besonders bevorzugt 4 bis 10 Gew.-% saurer Katalysator, bezogen auf Glukose, zugesetzt werden und noch 10 bis 600, bevorzugt 20 bis 300, besonders bevorzugt 30 bis 100 Minuten gerührt wird.

Das Lösen der Glukose dauert nicht länger als 60 Minuten. Die Glukose ist bei 80°C 30 Minuten nach Zugabe des Ionentauschers zu ca. 90 %, nach 90 Minuten zu ca. 95 % und nach 180 Minuten zu ca. 97 % umgesetzt.

Als saure Katalysatoren können alle löslichen Säuren, bzw. Schwefelsäure, Toluolsulfonsäure, Salzsäure und Methansulfonsäure und alle sulfonsauren Ionentauscher auf Phenol-Formaldehyd-Basis und auf Styrol-Divinylbenzolbasis eingesetzt werden, wie sie z.B. unter der Bezeichnung Lewatit oder Dowex im Handel sind. Bevorzugt werden heterogene Katalysatoren eingesetzt. Bevorzugt werden die heterogenen Katalysatoren in trockener Form eingesetzt. Weiterhin sind auch anorganische saure Ionenaustauscher, wie z.B. Zeolithe, insbesondere Zeolith Y, Schichtsilikate, wie z.B. Montmorillonit geeignet. Der pK_{A}-Wert der sauren Substanzen in Eisessig sollte Kleiner 0, bevorzugt kleiner -2 sein.

Die Aufarbeitung des Reaktionsgemisches erfolgt im wesentlichen durch Abfiltrieren des Katalysators und Abdestillieren des überschüssigen Alkohols, bevorzugt im Vakuum.

Zum Abdestillieren des überschüssigen Alkohols wird bevorzugt ein Dünnfilmverdampfer eingesetzt. Wird zum Abdestillieren ein Vakuum angelegt, liegt dies zwischen 0,1 und 100 mbar, bevorzugt zwischen 0,5 und 50 mbar, besonders bevorzugt zwischen 1 und 10 mbar.

Als Reaktoren zur Durchführung der Synthese kommen kontinuierlich und diskontinuierlich betriebene Kessel sowie sogenannte Gegenstromkolonnen in Frage. Prinzipiell können alle bekannten Reaktorsysteme zur Umsetzung flüssiger Stoffe benutzt werden.

Chlorethylglucopyranose kann vielfältig, z.B. zum Hydrophilisieren von Polymeren, eingesetzt werden.

Besonderer Vorteil des neuen Verfahrens sind die geringen Katalysatormengen, die kurze Reaktionszeit und die relativ geringe Produktverfärbung bei hohem Glucoseumsatz.

### Beispiel 1

1,8 kg (10 Mol) Glukose werden in 3,6 kg (45 Mol) Chlorethanol suspendiert und rasch unter Rühren auf 110°C aufgewärmt. Nach weniger als 40 Minuten ist die gesamte Zuckermenge in Lösung gegangen.

Die Temperatur der Reaktionsmischung wird durch Abdestillieren einer Chlorethanol-H₂O-Mischung auf 80°C gesenkt, hierzu wird der Druck in der Anlage schrittweise heruntergenommen.

Die Apparatur wird mit Stickstoff belüftet und 90 g trockener Lewatit SC 102 zugesetzt (Ionenaustauscher auf Basis von mit Divinylbenzol vernetztem sulfoniertem Polystyrol.

30 Minuten nach Zugabe des Katalysators zeigt das Gaschromatogramm einer silylierten Probe ca. 9,8 % Glukose und 88,6 % Chlorethylglucopyranose an, nach 90 Minuten sind es ca. 5,7 % Glukose und 92,5 % Chlorethylglucopyranose.

Die Reaktionslösung ist farblos bis leicht gelblich.

### Vergleichsbeispiel

180 g (1 Mol) Glukose, 9 g Lewatit SC 102 und 360 g (4.5 Mol) Chlorethanol werden unter Rühren suspendiert und auf 80°C erwärmt.

Nach 6 Stunden enthält die gelbe Mischung laut gaschromatographischer Analyse immer noch 25 % unumgesetzte Glukose.

## Patentansprüche

1. Verfahren zur Herstellung eines Gemisches, im wesentlichen bestehend aus α- und β-Chlorethylglucopyranose durch Umsetzung von Chlorethanol mit Glukose, dadurch gekennzeichnet, daß in 2 bis 10 Molen Chlorethanol 1 Mol Glukose bei 100 bis 130°C in Abwesenheit von Katalysatoren vorgelöst wird, wobei gegebenenfalls Wasser abdestilliert werden kann und anschließend bei 60 bis 90°C 1 bis 30 Gew.-% saurer Katalysator, bevorzugt saure anorganische und/oder organische Ionenaustauscher, bezogen auf eingesetzte Glukose, zugesetzt werden und 10 bis 600 Minuten nachgerührt wird.

2. Verfahren nach Anspruch 1, dadurch gekennzeichnet, daß anschließend der Katalysator abfiltriert und der Überschuß Chlorethanol im Vakuum abgezogen wird.

3. Verfahren nach Anspruch 2, dadurch gekennzeichnet, daß Vakua zwischen 0,1 und 100 mbar am Ende eingestellt werden.

4. Verfahren nach Anspruch 3, dadurch gekennzeichnet, daß ein Dünnfilmverdampfer eingesetzt wird.
